# DEMANDE DE BREVET EUROPEEN

(11) **EP 4 414 995 A1**
(43) Date de publication de la demande: **14.08.2024**
(21) Numéro de dépôt: 23155506.1
(22) Date de dépôt: 08.02.2023
(51) Int. Cl.: G16H 15/00

(54) **PROCÉDÉ DE GÉNÉRATION DE COMPTES-RENDUS D'UN ACTE MÉDICAL**

(71) Demandeur: VULGAROO, 33000 Bordeaux (FR)
(72) Inventeur: LELONG, Christophe, 33000 BORDEAUX (FR)
(74) Mandataire: Aquinov

(57) **Abrégé**

L'invention concerne un procédé de génération d'un compte-rendu d'un acte médical réalisé par un professionnel de la santé sur un patient ; le procédé étant mis en oeuvre par un système informatique comportant un premier terminal informatique, un serveur de traitement et un deuxième terminal informatique, le procédé comportant les étapes suivantes : Génération d'un premier compte-rendu, dit expert, de l'acte médical sur le premier terminal informatique ; Génération automatique, par le serveur de traitement, d'au moins un deuxième compte-rendu, dit simplifié, à partir du premier compte-rendu ; Transmission, par le serveur de traitement, des premier et deuxième comptes-rendus au moins au premier terminal informatique ; Génération, par le serveur de traitement et/ou par le premier terminal, d'un identifiant numérique associé au patient et audit acte médical, permettant au patient d'accéder auxdits premier et deuxième comptes-rendus via le deuxième terminal informatique.

## Description

### Domaine technique

L'invention se rapporte au domaine technique de la santé, et plus précisément à la production de comptes-rendus médicaux.

### Etat de la technique

De façon classique, lors d'un parcours de soins d'un patient, un ou plusieurs examens médicaux lui sont prescrits, comme une consultation d'un spécialiste, un examen médical faisant intervenir de l'imagerie ou un examen biologique. Le praticien conduisant l'examen rédige généralement un rapport ou un compte-rendu de l'examen médical, éventuellement muni d'observations, ou de conclusions de diagnostic. Ce rapport est alors transmis, soit informatiquement via le dossier médical du patient, soit directement via le patient, au praticien suivant du parcours de soins, et/ou au médecin coordonnant le parcours de soins.

Si cette méthode permet effectivement aux différents intervenants médicaux d'avoir accès aux résultats des examens précédents et de suivre efficacement le patient lors du parcours de soins, elle a pour inconvénient d'exclure le patient de la stratégie thérapeutique du parcours de soin.

En effet, un compte-rendu médical contient généralement des termes techniques médicaux complexes que le patient peut ne pas connaître. Par ailleurs, les réponses et conclusions apportées par le praticien dans le compte-rendu sont difficilement interprétables par le patient, par exemple parce que le praticien emploie des termes cliniques ou des acronymes, ou encore parce qu'il fait référence à des valeurs sans qu'un référentiel d'explication ne soit fourni.

Le patient peut ainsi perdre confiance dans la stratégie thérapeutique qui lui a été proposé, et réduire son observance et sa participation dans le parcours de soins, ce qui peut provoquer un échec de ce parcours de soins.

Il existe ainsi un besoin pour une solution permettant l'échange de comptes-rendus médicaux entre les différents praticiens d'un parcours de soins et le patient, selon des niveaux de compréhension adaptés.

L'invention se place donc dans ce contexte et cherche à résoudre l'ensemble des inconvénients précités en répondant audit besoin.

### Présentation de l'invention

A ces fins, l'invention a pour objet un procédé de génération de comptes-rendus d'un acte médical réalisé par un professionnel de la santé sur un patient ; le procédé étant mis en oeuvre par un système informatique comportant un premier terminal informatique, un serveur de traitement et un deuxième terminal informatique, le procédé comportant les étapes suivantes :
a. Génération d'un premier compte-rendu, dit expert, de l'acte médical sur le premier terminal informatique ;
b. Génération automatique, par le serveur de traitement, d'au moins un deuxième compte-rendu, dit simplifié, à partir du premier compte-rendu ;
c. Transmission, par le serveur de traitement, des premier et deuxième comptes-rendus au moins au premier terminal informatique ;
d. Génération, par le serveur de traitement et/ou par le premier terminal, d'un identifiant numérique associé au patient et audit acte médical, permettant au patient d'accéder auxdits premier et deuxième comptes-rendus via le deuxième terminal informatique.

L'invention propose ainsi par exemple que, lors de l'acte médical, un professionnel de la santé rédige le compte-rendu médical expert au moyen d'un terminal informatique relié à un serveur de traitement. L'acte médical pourra être un acte à des fins d'investigation ou de diagnostic, et comporter une anamnèse, une inspection, une palpation, une auscultation, un prélèvement, un examen faisant intervenir de l'imagerie médicale, ou encore une analyse d'un prélèvement et/ou d'une image médicale. L'acte médical pourra également être un acte à des fins thérapeutiques, et comporter un entretien oral visant à délivrer des conseils thérapeutiques, l'administration de médicaments, une intervention chirurgicale, radiologique, ou physique. Tout ou partie de l'acte médical pourra être pratiqué par des médecins de différentes spécialités médicales, notamment un chirurgien, un psychologue, un cardiologue, un oncologue, un radiologue ; par des dentistes, par des infirmiers, des secrétaires médicaux, des pharmaciens, des kinésithérapeutes, des biologistes médicaux, des techniciens de laboratoire, des aides-soignants, des ambulanciers.

Le premier compte-rendu peut ainsi être transmis depuis le terminal informatique au serveur de traitement, par exemple par un protocole de communication sans-fil, ou être directement rédigé sur une application logicielle du serveur de traitement accessible depuis le terminal informatique. Le premier compte-rendu est ainsi disponible et accessible depuis d'autres terminaux informatiques autorisés à se connecter au serveur de traitement, par exemple pour permettre à un autre professionnel de la santé, comme un autre praticien, au médecin traitant ou d'autres intervenants du parcours de soin de prendre connaissance de ce compte-rendu.

Parallèlement, le serveur de traitement peut alors générer un ou plusieurs comptes-rendus supplémentaires, à partir du premier compte-rendu, chaque compte-rendu généré correspondant à un niveau de compréhension simplifié donné. A la suite de la génération de ce ou ces comptes-rendus simplifiés, le serveur de traitement peut alors transmettre sur le premier terminal informatique l'ensemble des comptes-rendus ainsi qu'un identifiant numérique, comme un QR code, contenant un lien qui, lorsqu'il est scanné au moyen d'un smartphone formant le deuxième terminal informatique, permet au patient de télécharger les premier et deuxième comptes-rendus sur une application logicielle installée sur ce smartphone. On notera que l'identifiant numérique pourra être généré par le premier terminal informatique lui-même. Cet identifiant numérique peut ainsi être communiqué, si nécessaire, par le professionnel de la santé au patient, soit en lui montrant un moniteur du premier terminal informatique sur lequel est affiché cet identifiant numérique, soit en imprimant un support, comme une ordonnance, comportant cet identifiant numérique.

Le premier compte-rendu expert et chacun des comptes-rendus simplifiés peuvent ainsi être transmis au deuxième terminal informatique pour y être lu par le patient. Le patient dispose ainsi de différents comptes-rendus du même acte médical, associés chacun à un niveau de compréhension différent, de sorte qu'on améliore les chances que le patient comprenne le contenu du premier compte-rendu, et reste ainsi impliqué dans le parcours de soin.

### Définitions

Dans la présente invention, on entend par « terminal informatique », sans que cela soit limitatif, un ordinateur de bureau, un ordinateur portable, un serveur, une tablette tactile, un smartphone, un téléphone cellulaire et/ou un équipement d'un réseau informatique.

De préférence, chaque terminal informatique est équipé d'une unité de communication apte à échanger des données avec une unité de communication du serveur de traitement, notamment selon un ou plusieurs protocoles de communication sans-fil.

Si on le souhaite, le premier terminal informatique pourra être relié à ou faire partie d'un ou plusieurs systèmes informatiques hospitaliers pour recevoir des données relatives à l'acte médical via d'autres éléments de ce système, ledit système informatique pouvant par exemple être un système d'information radiologique (RIS) et/ou un système d'archivage et de transmission d'images (PACS).

Dans la présente invention, le serveur de traitement pourra faire partie du système informatique hospitalier dont fait partie le premier terminal informatique. Le cas échéant, le procédé selon l'invention pourra être mis en oeuvre au moyen de programmes ou d'applications logicielles dont une partie pourra être installée sur le premier terminal informatique, notamment aux fins de la génération du premier compte-rendu. Le premier compte-rendu sera ainsi transmis, une fois généré, au serveur de traitement.

En variante, le serveur de traitement pourra être un serveur distant d'un tel système informatique hospitalier, les services dudit serveur de traitement étant alors accessibles par le premier terminal informatique au moyen de méthodes de type cloud computing. Le cas échéant, l'ensemble des programmes ou applications logicielles nécessaires à la mise en oeuvre du procédé selon l'invention pourront être installés sur le serveur de traitement, tout ou partie de ces programmes ou applications logicielles pouvant être accessibles depuis le premierterminal informatique via des protocoles de communication sans-fil, comme par exemple au travers d'un navigateur internet et de protocole HTTPS et IP. Le premier compte-rendu sera ainsi généré directement sur le serveur distant.

Dans ce cas, on pourra prévoir que le procédé selon l'invention comporte une étape préalable d'identification et/ou d'authentification de la personne et/ou de l'application en charge de la génération du premier compte-rendu, notamment au travers d'un procédé d'authentification forte mis en oeuvre par le serveur de traitement ou un autre serveur dédié à ce procédé.

### Modes de réalisation

Dans un mode de réalisation de l'invention, l'étape de génération du premier compte-rendu comporte :
a. une étape de sélection sur le premier terminal informatique d'un modèle de compte-rendu dans une bibliothèque de modèles de comptes-rendus prédéterminés ;
b. une étape d'exécution sur le premier terminal informatique d'un questionnaire médical associé au modèle de compte-rendu sélectionné ;
c. et pour chaque question du questionnaire, une étape de fourniture d'au moins une réponse, via le premier terminal informatique, le premier compte-rendu comportant au moins l'ensemble des réponses fournies lors de l'exécution du questionnaire.

Par exemple, le premier compte-rendu pourra comporter uniquement l'ensemble des réponses fournies lors de l'exécution du questionnaire, chaque réponse étant alors associée à une question donnée du questionnaire, ou encore l'ensemble des paires de question-réponse fournies lors de l'exécution du questionnaire.

De préférence, ladite bibliothèque de modèles pourra être stockée dans une mémoire du serveur de traitement ou du premier terminal informatique, et l'étape de sélection pourra être mise en oeuvre au travers d'une application logicielle accessible depuis le premier terminal informatique sur laquelle est affichée ladite bibliothèque, l'application logicielle étant munie d'une interface de recherche et/ou de sélection d'un modèle.

Par exemple, le procédé pourra comporter une étape préalable d'identification sur une application logicielle, via le premier terminal informatique, d'une ou plusieurs caractéristiques de l'acte médical, comme une pathologie, un type d'examen, une spécialité du praticien ou une modalité d'imagerie médicale. Le cas échéant, l'étape de sélection d'un modèle de compte-rendu pourra comporter l'affichage dans ladite application logicielle d'un ou plusieurs modèles de la bibliothèque présélectionnés selon ladite ou lesdites caractéristiques identifiées.

L'étape d'exécution dudit questionnaire médical pourra comporter l'affichage du questionnaire sur une application logicielle accessible depuis le premier terminal informatique, ou en variante la lecture par dudit questionnaire par une application logicielle accessible depuis le premier terminal informatique et munie de moyens de synthèse vocale.

Dans un exemple de réalisation, le questionnaire médical pourra être un questionnaire séquentiel dynamique. En d'autres termes, les questions du questionnaire pourront être exécutées séquentiellement, les unes après les autres ou par groupes successifs, l'exécution d'une question ou d'un groupe de questions dépendant des réponses fournies à tout ou partie des questions ou groupes de questions précédents.

La fourniture des réponses aux questions pourra être réalisée manuellement par une personne, notamment via un clavier du premier terminal informatique. En variante, les réponses pourront être dictées au premier terminal informatique, via un microphone, le premier terminal informatique étant muni d'une application logicielle de dictée et de reconnaissance vocale.

On pourra prévoir que les réponses soient fournies par une ou plusieurs personnes, voire par un ou plusieurs algorithmes d'apprentissage automatique, ou machine learning.

Le premier compte-rendu pourra ainsi comporter l'ensemble des paires de question-réponse fournies lors de l'exécution du questionnaire, ces paires pouvant être réordonnées et/ou groupées en sections dans le premier compte-rendu. Cet ensemble pourra éventuellement être assorti d'un jeu de conclusions médicales.

Dans un autre mode de réalisation de l'invention, on pourra prévoir que le premier compte-rendu soit rédigé manuellement par un professionnel de la santé, identique ou distinct de celui ayant réalisé l'acte médical, puis scanné au moyen du premier terminal informatique. En variante, on pourra prévoir que le premier compte-rendu soit rédigé directement sur le premier terminal informatique par un professionnel de la santé, identique ou distinct de celui ayant réalisé l'acte médical, par exemple via un éditeur de texte ou de compte-rendu médical.

Dans un mode de réalisation de l'invention, le serveur de traitement comporte une mémoire dans laquelle sont stockés, pour chaque modèle de compte-rendu de la bibliothèque, au moins un jeu prédéfini d'une pluralité de règles de transformation d'une réponse dudit compte-rendu vers une réponse et/ou au moins une grille prédéfinie de sélection de réponses parmi l'ensemble des réponses dudit compte-rendu. Le cas échéant, l'étape de génération automatique du deuxième compte-rendu comporte :
a. une étape de sélection, par le serveur de traitement dans sa mémoire, de la grille de sélection et/ou du jeu de règles de transformation associés au modèle du premier compte-rendu ;
b. une étape d'application, par le serveur de traitement, de la grille de sélection et/ou du jeu de règles de transformation sélectionnés au premier compte-rendu pour générer le deuxième compte-rendu.

On pourra prévoir que chaque règle de transformation soit une règle de transformation d'une réponse associée à une question donnée vers une autre réponse associée à cette question donnée, une règle de transformation d'une paire de question-réponse vers une autre paire de question-réponse, ou encore une règle de transformation d'une réponse associée à une question donnée en une autre paire de question-réponse. De façon équivalente, on pourra prévoir que la grille de sélection soit une grille de sélection de réponses parmi l'ensemble des réponses dudit compte-rendu, ou une grille de sélection de paires de question-réponse parmi l'ensemble des paires de question-réponse dudit compte-rendu.

Ce jeu de règles et cette grille de sélection permettent ainsi de générer un deuxième compte-rendu adapté à un niveau de compréhension plus faible que le premier compte-rendu, en simplifiant le vocabulaire ou la syntaxe employée et en supprimant des réponses ou des paires de question-réponse qui n'apportent pas d'informations utiles ou essentielles au patient. Chaque jeu et chaque grille de sélection pourront par exemple être définis au préalable par des patients experts. On pourra indifféremment prévoir que le serveur de traitement applique seulement une grille de sélection, sans appliquer de règles de transformation, ou que le serveur de traitement applique seulement des règles de transformation, sans appliquer de grille de sélection, ou encore que le serveur de traitement applique conjointement des règles de transformation et une grille de sélection.

Avantageusement, l'étape de génération du premier compte-rendu comporte, pour chaque question du questionnaire, une étape de fourniture d'au moins une réponse, via le premier terminal informatique, pour former une paire de question-réponse, le premier compte-rendu comportant l'ensemble des questions-réponses fournies lors de l'exécution du questionnaire ; et, pour chaque modèle de compte-rendu de la bibliothèque, sont stockés dans la mémoire du serveur de traitement : au moins un jeu prédéfini d'une pluralité de règles de transformation d'une paire de question-réponse dudit compte-rendu vers une autre paire de question-réponse et/ou au moins une grille prédéfinie de sélection de paires de question-réponse parmi l'ensemble des paires de question-réponse dudit compte-rendu.

Avantageusement, chaque règle d'un jeu de règles de transformation associé à un modèle de compte-rendu est apte à remplacer, dans au moins une paire de question-réponse dudit compte-rendu, au moins une entrée donnée de la question et/ou de la réponse de ladite paire par une autre entrée prédéterminée.

On entend par « entrée » un terme, une proposition composée de plusieurs termes, une valeur ou un booléen de type « OUI » ou « NON ». En d'autres termes, il est possible de transformer tout ou partie d'une question et/ou d'une réponse pour simplifier le vocabulaire employé, pour transformer des valeurs en des termes relatifs, transformer une paire de question-réponse en proposition booléenne ou inversement, inverser une syntaxe grammaticale, etc. On pourra également envisager de synthétiser plusieurs paires de question-réponse en une seule question-réponse plus simple.

Le cas échéant, un jeu de règles de transformation associé à un modèle de compte-rendu pourra comporter au moins une règle apte à remplacer, dans une conclusion dudit compte-rendu, au moins une entrée donnée de ladite conclusion par une autre entrée prédéterminée.

Avantageusement, une grille de sélection associée à un modèle de compte-rendu est apte à conserver ou supprimer chaque paire de question-réponse dudit compte-rendu en fonction de la question et/ou de la réponse de ladite paire. Par exemple, une paire de question-réponse pourra être conservée selon que la réponse fournie à une question donnée soit positive ou négative, ou selon que la valeur fournie soit en dehors ou dans une plage prédéfinie.

Dans un mode de réalisation de l'invention, pour chaque modèle de compte-rendu de la bibliothèque, sont stockés dans la mémoire du serveur de traitement au moins un jeu prédéfini d'une pluralité de règles de transformation et/ou au moins une grille prédéfinie de sélection associée à un premier niveau de compréhension et au moins un jeu prédéfini d'une pluralité de règles de transformation et/ou au moins une grille prédéfinie de sélection associé à un deuxième niveau de compréhension. Le cas échéant, l'étape de génération automatique comporte :
a. une étape de sélection, par le serveur de traitement dans sa mémoire, de la grille de sélection et/ou du jeu de règles de transformation associés au modèle du premier compte-rendu et au premier niveau de compréhension et de la grille de sélection et/ou du jeu de règles de transformation associés au modèle du premier compte-rendu et au deuxième niveau de compréhension ;
b. une étape d'application au premier compte-rendu, par le serveur de traitement, de la grille de sélection et/ou du jeu de règles de transformation associés au premier niveau de compréhension pour générer le deuxième compte-rendu et de la grille de sélection et/ou du jeu de règles de transformation associés au deuxième niveau de compréhension pour générer un troisième compte-rendu ; et en ce que l'étape de transmission comporte la transmission, par le serveur de traitement, des premier, deuxième et troisième comptes-rendus au moins au premier terminal informatique.

On comprend ainsi qu'on peut générer plusieurs comptes-rendus d'un même acte médical selon différents niveaux de compréhension, de sorte à permettre au patient de progresser dans sa compréhension du parcours de soins qu'il suit.

Par exemple, on pourra prévoir que le deuxième compte-rendu soit un compte-rendu vulgarisé détaillé, comprenant toutes les réponses ou les paires de question-réponse du premier compte-rendu, et dans lesquelles les termes techniques et/ou médicaux sont remplacés par des termes courants ; et que le troisième compte-rendu soit un compte-rendu vulgarisé abrégé, comprenant seulement des réponses ou des paires de question-réponse du premier compte-rendu qui ont été jugées essentielles pour le patient et dans lesquelles les termes techniques et/ou médicaux sont remplacés par des termes courants.

Dans un autre mode de réalisation de l'invention, alternatif ou cumulatif, le serveur de traitement comporte une mémoire dans laquelle sont stockés au moins un lexique comportant une pluralité de mots-clés prédéterminés et au moins un dictionnaire associant à chaque mot-clé du lexique une définition dudit mot-clé. Le cas échéant, l'étape de génération automatique du deuxième compte-rendu peut comporter :
a. une étape d'identification, par le serveur de traitement, d'un ou plusieurs mots-clés dans le premier compte-rendu à partir dudit lexique ;
b. pour chaque mot-clé identifié, une étape de sélection, par le serveur de traitement, d'une définition associée audit mot-clé identifié dans le dictionnaire ;
c. une étape d'insertion, par le secteur de traitement, de chaque définition sélectionnée dans le premier compte-rendu pour générer le deuxième compte-rendu.

Le deuxième compte-rendu correspond ainsi au premier compte-rendu, enrichi des définitions de termes médicaux, scientifiques ou techniques, qui permettent de faciliter la compréhension du patient. Ce mode de réalisation est particulièrement adapté au cas où le premier compte-rendu est généré sans faire appel à un questionnaire associé à un modèle de compte-rendu préétabli, et notamment lorsqu'il est saisi librement par le professionnel de la santé via un éditeur de texte ou lorsqu'il est rédigé manuellement ou encore lorsqu'il est dicté.

De préférence, l'étape de génération pourra comprendre, préalablement à l'étape d'identification de mots clés, une étape d'extraction de texte à partir du premier compte-rendu et/ou une étape de reconnaissance de texte dans le premier compte-rendu, notamment mise en oeuvre au moyen de méthodes de reconnaissance optique de caractères. Le cas échéant, l'étape d'identification de mots clés sera mise en oeuvre à partir des textes extraits du premier compte-rendu et/ou reconnus dans le premier compte-rendu.

Avantageusement, l'étape d'identification de mots-clés pour comprendre la comparaison de chacun des termes du premier compte-rendu avec les mots-clés du lexique et/ou la recherche de chacun des mots-clés du lexique dans les termes du premier compte-rendu. Le cas échéant, ladite comparaison et/ou ladite recherche pourra s'étendre à l'identification de termes apparentés et/ou similaires auxdits mots-clés et/ou de synonymes desdits mots-clés.

Avantageusement, chaque définition sélectionnée pourra être insérée sous la forme d'une note de bas de page du deuxième compte-rendu ou dans une annexe du deuxième compte-rendu, et chaque mot-clé identifié dans le premier compte-rendu pourra être assorti, dans le deuxième compte-rendu, d'un renvoi vers cette définition. En variante, chaque mot-clé identifié dans le premier compte-rendu pourra être transformé, dans le deuxième compte-rendu, en un hyperlien permettant d'accéder à la définition sélectionnée dudit mot-clé. En variante encore, chaque mot-clé identifié dans le premier compte-rendu pourra être assorti d'un écouteur logiciel, ou listener, d'un évènement donné, comme un clic du mot-clé ou un survol du mot-clé. Le cas échéant, le déclenchement dudit évènement donné par le patient, via le deuxième terminal informatique, pourra entraîner l'affichage de la définition sélectionnée dudit mot-clé, par exemple dans une info-bulle.

Avantageusement, sont stockés dans la mémoire du serveur de traitement au moins un premier dictionnaire associé à un premier niveau de compréhension et au moins un deuxième dictionnaire associé à un deuxième niveau de compréhension. Le cas échéant, l'étape de sélection comporte, pour chaque mot-clé identifié, la sélection d'une définition associée audit mot-clé dans le premier dictionnaire et dans le deuxième dictionnaire ; et l'étape d'insertion comporte l'insertion dans le premier compte-rendu de chaque définition sélectionnée dans le premier dictionnaire pour générer le deuxième compte-rendu et l'insertion dans le premier compte-rendu de chaque définition sélectionnée dans le deuxième dictionnaire pour générer un troisième compte-rendu ; l'étape de transmission comporte la transmission, par le serveur de traitement, des premier, deuxième et troisième comptes-rendus au moins au premier terminal informatique. On pourra éventuellement prévoir également d'autres dictionnaires permettant d'obtenir, pour un mot-clé d'une langue donnée, une définition dudit mot-clé dans une langue différente.

On comprend ainsi qu'on peut générer plusieurs comptes-rendus d'un même acte médical selon différents niveaux de compréhension, de sorte à permettre au patient de progresser dans sa compréhension du parcours de soins qu'il suit.

Dans les exemples qui précèdent, les niveaux de compréhension pourront par exemple être définis selon des tests de lisibilité Flesch-Kincaid ou par tout méthode d'évaluation de la compréhension adaptée au domaine des comptes-rendus médicaux.

Dans un mode de réalisation de l'invention, le procédé comporte, ultérieurement à la génération de l'identifiant numérique :
a. une étape de saisie par le patient, via le deuxième terminal informatique, dudit identifiant numérique ;
b. à la suite de ladite saisie ; une étape de transmission par le serveur de traitement des premier et deuxième comptes-rendus au deuxième terminal informatique.

Avantageusement, le deuxième terminal informatique peut être équipé d'une application logicielle comportant une interface de sélection d'un niveau de compréhension de l'utilisateur du deuxième terminal informatique. Le cas échéant, le procédé comporte une étape ultérieure d'affichage, sur ladite application logicielle, du premier compte-rendu et/ou du deuxième compte-rendu selon le niveau de compréhension sélectionné par l'utilisateur du deuxième terminal informatique au moyen de ladite interface de sélection. Le patient peut ainsi lire l'un ou l'autre des comptes-rendus et vérifier ainsi quel est son niveau de compréhension et l'améliorer.

Avantageusement toujours, ladite application logicielle peut comporter une interface de confirmation de compréhension par l'utilisateur du deuxième terminal informatique. Le cas échéant, le procédé comporte :
a. A la suite de l'affichage, sur ladite application logicielle, du premier compte-rendu et/ou du deuxième compte-rendu, une étape d'affichage, sur ladite application logicielle, de ladite interface de confirmation de compréhension ;
b. A la suite d'une saisie d'informations par l'utilisateur du deuxième terminal informatique, via ladite interface de confirmation de compréhension, la transmission desdites informations saisies au serveur de traitement et/ou au premier terminal informatique.

Le patient peut ainsi confirmer qu'il a lu et compris l'un des comptes-rendus, ou au contraire qu'aucun des comptes-rendus ne lui a permis de comprendre les résultats de l'acte médical, ou encore que les comptes-rendus manquent de clarté et qu'une partie du compte-rendu n'est pas claire. Ces informations sont alors transmises au serveur de traitement, ou au premier terminal informatique, pour que le praticien en charge de l'acte médical ou un autre intervenant du parcours de soins en prenne connaissance et puisse préparer un entretien ultérieur avec le patient pour lui apporter des explications.

L'invention a également pour objet un programme d'ordinateur comprenant un code de programme qui est conçu pour mettre en oeuvre le procédé selon l'invention.

L'invention a également pour objet un support de données sur lequel est enregistré le programme d'ordinateur selon l'invention.

### Brève description des figures

D'autres avantages et caractéristiques de la présente invention sont maintenant décrits à l'aide d'exemples uniquement illustratifs et nullement limitatifs de la portée de l'invention, et à partir des figures annexées, dans lesquelles :
[Fig. 1] représente, schématiquement et partiellement, un système informatique pour la mise en oeuvre d'un procédé selon un exemple de réalisation de l'invention ;
[Fig. 2] représente, schématiquement et partiellement, un procédé de génération de comptes-rendus d'un acte médical selon un exemple de réalisation de l'invention, mis en oeuvre au moyen du système informatique de la [Fig. 1] ;
[Fig. 3] représente, schématiquement et partiellement, une vue d'un questionnaire médical affiché sur un terminal informatique du système de la [Fig. 1] lors de la mise en oeuvre d'une étape du procédé de la [Fig. 2] ;
[Fig. 4] représente, schématiquement et partiellement, un compte-rendu médical transmis à un serveur de traitement du système de la [Fig. 1] lors de la mise en oeuvre d'une autre étape du procédé de la [Fig. 2] ;
[Fig. 5] représente, schématiquement et partiellement, un jeu de règles employé par le serveur de traitement du système de la [Fig. 1] lors de la mise en oeuvre d'une autre étape du procédé de la [Fig. 2] ;
[Fig. 6] représente, schématiquement et partiellement, une grille de sélection employé par le serveur de traitement du système de la [Fig. 1] lors de la mise en oeuvre d'une autre étape du procédé de la [Fig. 2] ;
[Fig. 7] représente, schématiquement et partiellement, différents comptes-rendus médicaux générés par le serveur de traitement du système de la [Fig. 1] lors de la mise en oeuvre d'une autre étape du procédé de la [Fig. 2] ; et
[Fig. 8] représente, schématiquement et partiellement, une vue d'une interface d'une application logicielle affichée sur un terminal informatique du système de la [Fig. 1] lors de la mise en oeuvre d'une autre étape du procédé de la [Fig. 2].

Dans la description qui suit, les éléments identiques, par structure ou par fonction, apparaissant sur différentes figures conservent, sauf précision contraire, les mêmes références.

Les procédés qui vont être décrits peuvent également être mis en oeuvre par des programmes logiciels exécutables par un système informatique. En outre, leur mise en oeuvre pourra indifféremment être réalisé par un traitement distribué et/ou un traitement parallèle, notamment pour traiter en parallèle plusieurs données.

Les figures décrites dans le présent document sont destinées à fournir une compréhension générale de l'invention sous divers modes de réalisation. Ces figures ne sont pas destinées à servir de description complète de tous les éléments et caractéristiques des appareils, processeurs et systèmes nécessaires à l'invention. De nombreux autres modes de réalisation de l'invention, ou des combinaisons de ceux-ci peuvent être apparents pour l'homme du métier à la lecture de cette description, en combinant les modes de réalisation divulgués. D'autres modes de réalisation peuvent être dérivés de la description, de sorte que des substitutions et des changements structurels et logiques peuvent être effectués sans s'écarter du cadre de la présente invention.

En outre, la description et les figures doivent être considérées comme illustratives plutôt que restrictives, et les revendications annexées sont destinées à couvrir toutes les modifications, améliorations et autres modes de réalisation de l'invention. Ainsi, la portée des revendications suivantes doit être déterminée par l'interprétation la plus large possible des revendications et de leurs équivalents et ne doit pas être restreinte ou limitée par la description qui précède.

### Description des modes de réalisation

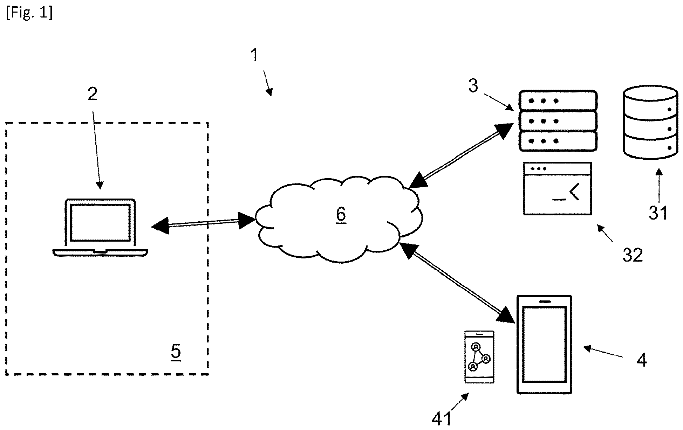

On a représenté en [Fig. 1] un système informatique 1 pour la mise en oeuvre d'un procédé de génération de comptes-rendus d'un acte médical selon un exemple de réalisation de l'invention.

Le système informatique 1 comporte un premier terminal informatique 2, un serveur de traitement 3 et un deuxième terminal informatique 4.

Dans l'exemple décrit, le premier terminal informatique 2 est un ordinateur de bureau, faisant partie d'un système d'information hospitalier 5, par exemple d'un hôpital, d'une clinique, d'une unité de radiologie, d'une unité de chirurgie, d'un cabinet de consultation ou d'un laboratoire d'analyse.

Le serveur de traitement 3 est distant du système d'information hospitalier. Il comporte une mémoire 31. Le premier terminal informatique 2, ou le système d'information hospitalier 5, et le serveur de traitement 3 sont chacun munis d'une unité de communication sans-fil, de sorte que les services offerts par le serveur de traitement 3 soient accessibles par le premier terminal informatique 2, notamment par un réseau de communication sans-fil 6 mettant en oeuvre des protocoles de communication de type HTTPS et IP.

Tel que ce sera décrit ultérieurement, une application logicielle 32 installée sur le serveur de traitement 3 peut notamment être exécutée sur le premier terminal informatique 2 au travers d'un navigateur internet.

Le deuxième terminal informatique 4 est, dans l'exemple décrit, un smartphone d'un patient pouvant recevoir, au travers d'une application logicielle 41 installée sur ce smartphone, des données transmises par le serveur de traitement 3.

On pourra prévoir en variante que le premier terminal informatique 2, le système d'information hospitalier 5 et le serveur de traitement 3 soient hébergés dans une même infrastructure, ces différents éléments pouvant alors être reliés les uns aux autres par un réseau de communication filaire et/ou un réseau de communication sans-fil.

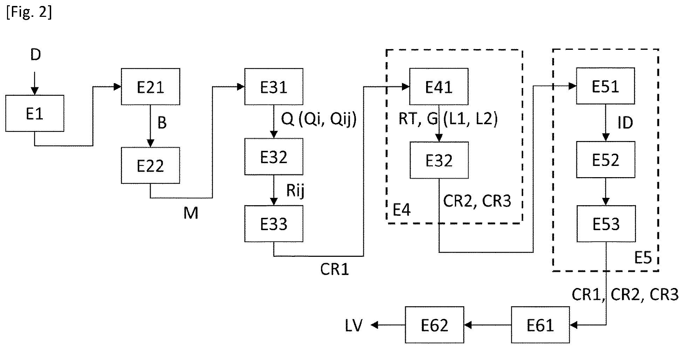

En lien avec la [Fig. 2], on va maintenant décrire un procédé de génération de comptes-rendus d'un acte médical réalisé par un professionnel de la santé sur un patient, et mis en oeuvre au moyen du système informatique 1.

Dans des étapes non représentées, le patient poursuit un parcours au sein de l'établissement hospitalier ou du laboratoire dans lequel est pratiqué l'acte médical. Ce parcours pourra inclure une étape d'accueil, une ou plusieurs étapes d'examen faisant notamment intervenir de l'imagerie médicale, des prélèvements, des analyses, de la chirurgie, des soins ou encore des entretiens avec des membres du personnel médical, et une étape de sortie. Un ensemble de données D pourra ainsi être collecté au fur et à mesure du parcours par différentes composantes du système d'information 5, ces composantes pouvant être du personnel médical, notamment du personnel d'accueil, un ambulancier, un médecin ou un infirmier, des équipements médicaux ou un système d'information connexe comme un système d'information radiologique (RIS), un système d'archivage et de transmission d'images (PACS), voire au travers de systèmes d'information externes, comme des services informatiques d'organismes publics.

Cet ensemble de données D pourra ainsi comprendre des informations issues d'un dossier patient informatisé, des données cliniques, des images médicales acquises selon une ou plusieurs modalités d'imagerie médicale.

Lors d'une des étapes du parcours du patient, une composante du système d'information 5 initie la mise en oeuvre du procédé selon l'invention. Dans l'exemple décrit, il s'agit du praticien ayant réalisé l'acte médical sur le patient. En variante, on pourra prévoir que le procédé soit initié par une composante non humaine du système d'information 5, notamment par un programme informatique, ou encore que le procédé soit mis en oeuvre par plusieurs composantes du système d'information 5, notamment par différents intervenants du personnel médical et à différents instants du parcours du patient, en amont comme en aval de l'acte médical.

Dans une première étape E1, le praticien se connecte via le premier terminal informatique 2 à l'application logicielle 32 du serveur distant 3, au travers d'un procédé d'authentification forte mis en oeuvre par un autre serveur dédié à ce procédé (non représenté).

Dans une deuxième étape E21, une bibliothèque B de modèles de comptes-rendus, stockée dans la mémoire 31 du serveur de traitement 3, est affichée dans l'application logicielle 32 sur le premier terminal informatique 2, avec une interface de recherche et de sélection d'un modèle. On pourra notamment prévoir que la bibliothèque B soit filtrée lors de l'affichage au moyen de tout ou partie des données de l'ensemble de données B. En variante, on pourra prévoir que la bibliothèque B soit stockée dans une mémoire d'un autre serveur du système informatique 1, le serveur de traitement 3 pouvant alors interroger ou accéder à cet autre serveur pour en extraire tout ou partie de cette bibliothèque B.

Dans une étape E22, le praticien sélectionne ainsi sur le premier terminal informatique 2 un modèle M de compte-rendu, par exemple en recherchant dans la bibliothèque B un modèle de compte-rendu associé au type d'acte médical qu'il a pratiqué, à la modalité d'imagerie médicale qu'il a employée et/ou à la pathologie qu'il a diagnostiquée.

Dans une étape E31, un questionnaire médical Q, stocké dans la mémoire 31 en étant associé au modèle M sélectionné, est exécuté sur l'application logicielle 32. En variante, on pourra prévoir que le questionnaire Q soit stocké dans une mémoire d'un autre serveur du système informatique 1, le serveur de traitement 3 pouvant alors interroger ou accéder à cet autre serveur pour en extraire ce questionnaire Q.

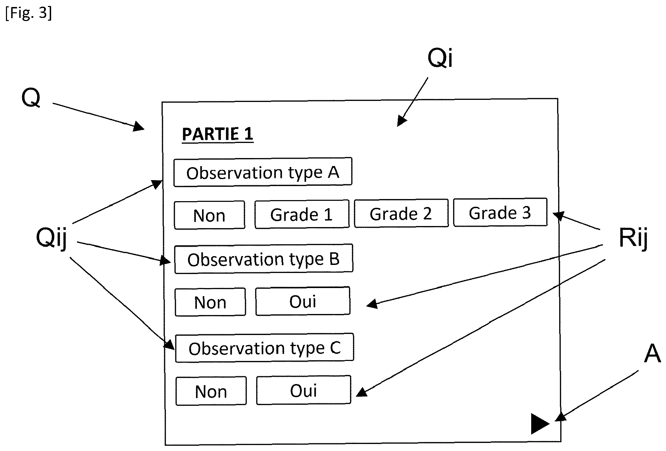

Comme représenté en [Fig. 3], dans l'exemple décrit, les questions Qij du questionnaire Q sont affichées par bloc ou section Qi sur le premier terminal informatique 2 via l'application logicielle 32, un composant d'action A permettant au praticien de naviguer d'un bloc à un autre sur l'application 32.

Chaque question Qij est affichée dans l'application logicielle 32 en étant muni d'un composant de réponse permettant au praticien de fournir une réponse Rij à cette question Qij dans une étape E32. De façon non limitative, chaque composant de réponse Rij pourra indifféremment comporter l'un ou plusieurs ou encore une combinaison de plusieurs des éléments suivants : un champ de saisie de texte, un bouton de sélection, une case à cocher, une liste de sélection déroulante, un curseur.

On pourra prévoir que le questionnaire médical Q soit dynamique, et que l'affichage d'une question Rij voire d'un bloc Qi dépendent des réponses fournies à tout ou partie des questions ou blocs de questions précédents, sans sortir du cadre de la présente invention.

On pourra également prévoir que tout ou partie des réponses Rij soient saisies par d'autres composantes, humaines, logicielles ou matérielles, du système d'information 5, au cours du parcours du patient, en amont et/ou pendant et/ou en aval de l'acte médical, notamment à partir de l'ensemble de données D.

Dans des variantes de réalisation de l'invention non représentées, le questionnaire Q pourra être exécuté, totalement ou partiellement, selon d'autres modalités, notamment en étant lu par des moyens de synthèse vocale du premier terminal informatique 2 et/ou du serveur de traitement 3. On pourra prévoir que les réponses Rij soient saisies manuellement par une personne, via un clavier et/ou une souris équipant le premier terminal informatique 2, ou soient dictées, via un microphone équipant le premier terminal informatique 2.

De façon non limitative, à l'issue de l'étape E32, le praticien peut être invité à fournir un ensemble de conclusions de diagnostic.

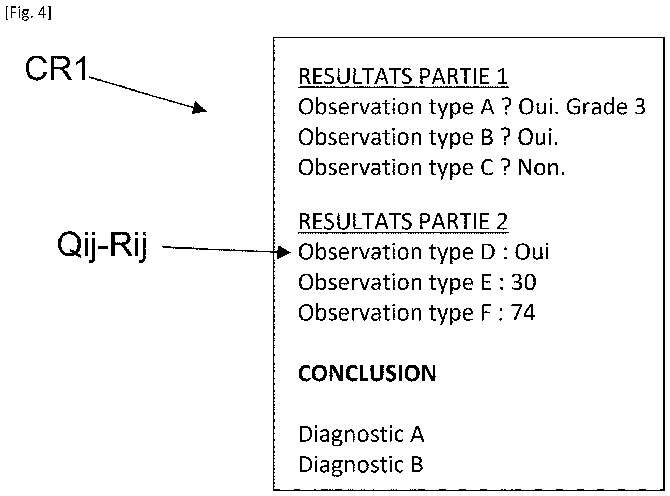

Dans une étape E33, l'ensemble des paires de question-réponse Qij-Rij fournies à l'issue des étapes E31 et E32, et le cas échéant les conclusions fournies, sont assemblées par le serveur de traitement 3 pour former un premier compte-rendu CR1, visible en [Fig. 4].

Dans une étape E4, d'autres comptes-rendus CR2, dit vulgarisé, et CR3, dit simplifié, sont générés par le serveur de traitement 3 à partir du premier compte-rendu CR1. On pourra prévoir que les différents comptes-rendus CR1, CR2 et CR3 soient générés simultanément à partir des paires de question-réponse Qij-Rij sans sortir du cadre de la présente invention.

A ces fins, pour chaque modèle M de compte-rendu de la bibliothèque B, sont stockés dans la mémoire 31 du serveur de traitement 3 au moins un jeu prédéfini d'une pluralité de règles de transformation RT et/ou au moins une grille prédéfinie de sélection G associé à un premier niveau de compréhension L1 et au moins un jeu prédéfini d'une pluralité de règles de transformation RT et/ou au moins une grille prédéfinie de sélection G associé à un deuxième niveau de compréhension L2.

Dans l'exemple décrit, un même jeu de règles de transformation RT est associé aux deux niveaux de compréhension L1 et L2 et aucune grille n'est associée au premier niveau L1 tandis qu'une grille G est associée au deuxième niveau L2. On pourra prévoir en variante que des jeux et des grilles différentes soient associés à chacun des niveaux L1 et L2, ou encore prévoir un nombre supérieur de niveaux de compréhension, sans sortir du cadre de la présente invention. Les niveaux de compréhension pourront être prédéterminés, ou au contraire choisis par le praticien.

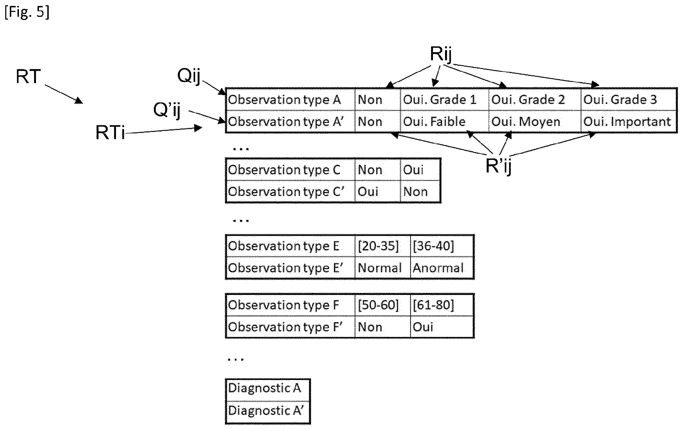

On a représenté schématiquement en [Fig. 5] un exemple de jeu de règles de transformation RT sous la forme d'une table de correspondance, ou lookup table, comprenant une pluralité de règles RTi.

Chaque règle RTi du jeu de règles de transformation RT associé au modèle M de compte-rendu permet ainsi de remplacer, dans chacune des paires de question-réponse Qij-Rij du premier compte-rendu CR1 et le cas échéant dans les conclusions, au moins une entrée donnée de la question Qij par une autre entrée prédéterminée pour obtenir une autre question Q'ij et au moins une entrée donnée de la réponse Rij fournie par une autre entrée prédéterminée pour obtenir une autre réponse R'ij.

Au regard de la [Fig. 5], on notera ainsi qu'un ou plusieurs termes, une valeur ou un booléen de type « OUI » ou « NON » d'une question Qij ou d'une réponse Rij peuvent être remplacés indifféremment par un ou plusieurs termes, une valeur ou un booléen de type « OUI » ou « NON », de sorte à obtenir une question Q'ij ou une réponse R'ij dont la syntaxe ou le vocabulaire est simplifiée, dont les valeurs sont transformées en termes relatifs, pour transformer une paire de question-réponse en proposition booléenne ou inversement, voire pour inverser une syntaxe grammaticale.

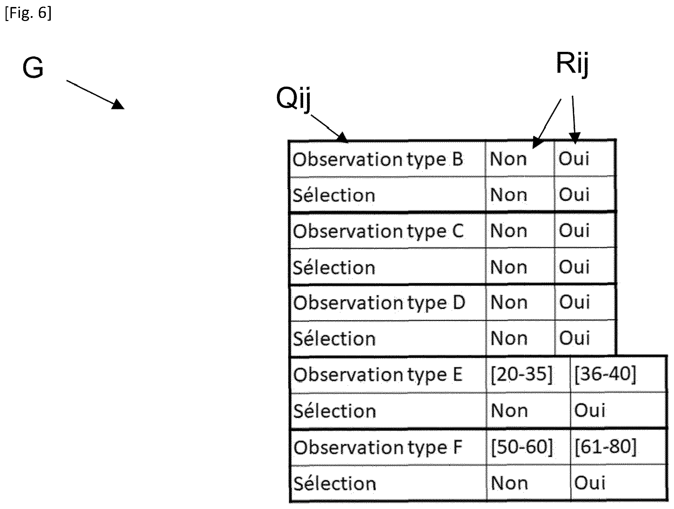

On a représenté schématiquement en [Fig. 6] un exemple de grille de sélection G sous la forme d'une table de correspondance.

Cette grille de sélection G associée au modèle M de compte-rendu permet ainsi de conserver ou supprimer certaines paires de question-réponse Qij-Rij du premier compte-rendu CR1 en fonction des réponses Rij.

Au regard de la [Fig. 5], on comprend que certaines des paires de question-réponse Qij-Rij sont conservées selon que la réponse Rij fournie à la question Qij est positive ou négative, ou selon que la valeur fournie soit est dans une plage ou une autre plage. On pourra prévoir qu'une réponse Rij fournie à une question Qij entraine la conservation ou la suppression d'une autre paire de question-réponse Qij-Rij ou encore que des réponses Rij fournies à plusieurs questions Qij entrainent la conservation ou la suppression d'une ou plusieurs de ces questions-réponses Qij-Rij.

Ainsi, dans une étape E41 de sélection, le serveur de traitement 3 sélectionne dans sa mémoire 31 les grilles de sélection G et/ou les jeux de règles de transformation RT associés au modèle M de compte-rendu sélection et aux différents niveaux de compréhension L1 et L2.

Puis, dans une étape E42 d'application, pour chacun des niveaux de compréhension L1 et L2, le serveur de traitement 3 applique la grille de sélection G et/ou le jeu de règles de transformation RT associés à ce niveau de compréhension pour générer un autre compte-rendu.

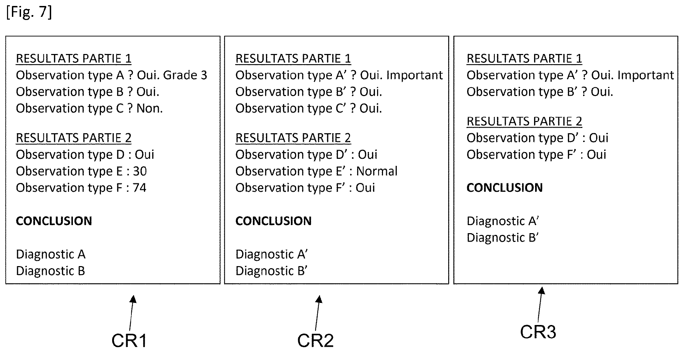

Dans l'exemple décrit, comme illustré en [Fig. 7], le serveur de traitement 3 génère donc, à l'issue de l'étape E42, un deuxième compte-rendu CR2 vulgarisé, comprenant des paires de question-réponse Q'ij-R'ij obtenues à partir de toutes les paires de question-réponse Qij-Rij du premier compte-rendu CR1 et du jeu de règles de transformation R. Ce deuxième compte-rendu CR2 correspond ainsi au premier compte-rendu CR1, dans lequel les termes techniques et/ou médicaux sont remplacés par des termes courants.

Le serveur de traitement 3 génère également, à l'issue de l'étape E42, un troisième compte-rendu CR3 simplifié, comprenant des paires de question-réponse Q'ij-R'ij obtenues à partir de seulement certaines des paires de question-réponse Qij-Rij du premier compte-rendu CR1, de la grille de sélection G et du jeu de règles de transformation R. Ce troisième compte-rendu CR3 comprend seulement des paires de question-réponse du premier compte-rendu CR1 qui ont été jugées essentielles pour le patient et dans lesquelles les termes techniques et/ou médicaux sont remplacés par des termes courants.

On notera que le premier compte-rendu CR1 est automatiquement associé à un niveau de compréhension L0.

Dans une étape E5, les premier, deuxième et troisième comptes-rendus CR1, CR2 et CR3 sont transmis par le serveur de transmission 3 aux premier et deuxième terminaux informatiques 2 et 4.

Plus précisément, dans une étape E51, le serveur de traitement 3 génère un identifiant numérique ID, par exemple sous la forme d'un QR code, qu'il transmet au premier terminal informatique 2, pour pouvoir être communiqué au patient. Ce QR code pourra indifféremment être scanné directement par le patient au moyen du smartphone 4 ou être imprimé par le praticien, par exemple sur une feuille d'ordonnance, pour pouvoir être scanné ultérieurement par le patient au moyen du smartphone 4. Parallèlement, les premier, deuxième et troisième comptes-rendus CR1, CR2 et CR3 sont transmis par le serveur de transmission 3 au premier terminal informatique 2, via l'application logicielle 32.

Dans une étape E52, le QR code ID est saisi par le patient sur le deuxième terminal informatique 4, par exemple en étant scanné via l'application logicielle 41 installée sur le smartphone 4.

Ce QR code permet ainsi au patient de télécharger automatiquement, dans une étape E53, les comptes-rendus CR1, CR2 et CR3 depuis le serveur de traitement 3 dans l'application logicielle 41 installée sur le smartphone 4.

Dans une variante non représentée, on pourra prévoir que les premier, deuxième et troisième comptes-rendus CR1, CR2 et CR3 soient transmis par le serveur de transmission 3 vers un autre serveur équipé d'une mémoire destinée à stocker des dossiers patients informatisés. Le patient pourra ainsi accéder à ces comptes-rendus en se connectant via le deuxième terminal informatique 4 à une application logicielle hébergée par cet autre serveur.

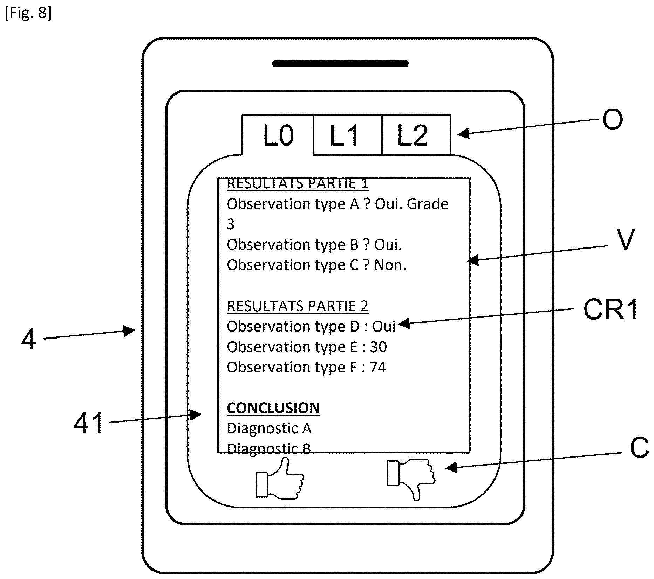

Comme représenté en [Fig. 8], l'application logicielle 41 comporte une interface de visualisation V desdits comptes-rendus CR1, CR2 et CR3 ainsi qu'une interface de sélection O des niveaux de compréhension L0, L1 et L2.

Le patient peut ainsi sélectionner, via l'interface de sélection O, le niveau de compréhension qui lui semble adapté, le compte-rendu correspondant étant ainsi affiché dans une étape E61 dans l'interface de visualisation V.

L'application logicielle comporte également une interface de confirmation C, affichée simultanément dans l'étape E61, et permettant au patient de préciser son niveau de compréhension LV du compte-rendu affiché dans cette interface de visualisation V, après sa lecture. On pourra prévoir que l'interface de confirmation C permette au patient une sélection plus fine de son niveau de compréhension que celle représentée en [Fig. 8] et/ou lui permette d'ajouter des commentaires, sans sortir du cadre de la présente invention.

Ainsi, à la suite d'une interaction entre le patient et l'interface de confirmation C, le niveau de compréhension sélectionné est transmis, dans une étape E62, au serveur de traitement 3, et éventuellement relayé au premier terminal informatique 2, de sorte que le professionnel de la santé ayant pratiqué l'acte médical puisse prévoir, le cas échéant, un entretien d'explication avec le patient. De façon alternative ou cumulative, les différents comptes-rendus CR1, CR2 et CR3 ainsi que le niveau de compréhension sélectionné pourront être transmis à un autre terminal informatique du système 1, notamment employé par un autre intervenant du parcours de soins poursuivi par le patient.

On notera qu'on pourra prévoir des aspects différents des différentes interfaces qui ont été représentées et/ou d'ajouter d'autres fonctionnalités, sans sortir du cadre de la présente invention.

La description qui précède explique clairement comment l'invention permet d'atteindre les objectifs qu'elle s'est fixée, à savoir proposer un procédé permettant l'échange de comptes-rendus médicaux entre les différents praticiens d'un parcours de soin et le patient, selon des niveaux de compréhension adaptés. Ces objectifs sont notamment atteints au moyen d'un serveur de traitement capable de générer, à partir d'un compte-rendu médical généré au niveau d'un premier terminal informatique, des comptes-rendus simplifiés, abrégés et/ou vulgarisés et de transmettre l'ensemble des comptes-rendus à un autre terminal informatique.

En tout état de cause, l'invention ne saurait se limiter aux modes de réalisation spécifiquement décrits dans ce document, et s'étend en particulier à tous moyens équivalents et à toute combinaison techniquement opérante de ces moyens.

## Revendications

1. Procédé de génération de comptes-rendus d'un acte médical réalisé par un professionnel de la santé sur un patient ; le procédé étant mis en oeuvre par un système informatique comportant un premier terminal informatique, un serveur de traitement et un deuxième terminal informatique, le procédé comportant les étapes suivantes :
a. Génération d'un premier compte-rendu, dit expert, de l'acte médical sur le premier terminal informatique ;
b. Génération automatique, par le serveur de traitement, d'au moins un deuxième compte-rendu, dit simplifié, à partir du premier compte-rendu ;
c. Transmission, par le serveur de traitement, des premier et deuxième comptes-rendus au moins au premier terminal informatique ;
d. Génération, par le serveur de traitement et/ou par le premier terminal, d'un identifiant numérique associé au patient et audit acte médical, permettant au patient d'accéder auxdits premier et deuxième comptes-rendus via le deuxième terminal informatique.

2. Procédé selon la revendication précédente, dans lequel l'étape de génération du premier compte-rendu comporte une étape de sélection sur le premier terminal informatique d'un modèle de compte-rendu dans une bibliothèque de modèles de comptes-rendus prédéterminés, une étape d'exécution sur le premier terminal informatique d'un questionnaire médical associé au modèle de compte-rendu sélectionné, et pour chaque question du questionnaire, une étape de fourniture d'au moins une réponse, via le premier terminal informatique, le premier compte-rendu comportant au moins l'ensemble des réponses fournies lors de l'exécution du questionnaire.

3. Procédé selon la revendication précédente, **caractérisé en ce que** le serveur de traitement comporte une mémoire dans laquelle sont stockés, pour chaque modèle de compte-rendu de la bibliothèque, au moins un jeu prédéfini d'une pluralité de règles de transformation d'une réponse dudit compte-rendu vers une autre réponse et/ou au moins une grille prédéfinie de sélection de réponses parmi l'ensemble des réponses dudit compte-rendu, et **en ce que** l'étape de génération automatique du deuxième compte-rendu comporte :
a. une étape de sélection, par le serveur de traitement dans sa mémoire, de la grille de sélection et/ou du jeu de règles de transformation associés au modèle du premier compte-rendu ;
b. une étape d'application, par le serveur de traitement, de la grille de sélection et/ou du jeu de règles de transformation sélectionnés au premier compte-rendu pour générer le deuxième compte-rendu.

4. Procédé selon la revendication précédente, dans lequel l'étape de génération du premier compte-rendu comporte, pour chaque question du questionnaire, une étape de fourniture d'au moins une réponse, via le premier terminal informatique, pour former une paire de question-réponse, le premier compte-rendu comportant l'ensemble des questions-réponses fournies lors de l'exécution du questionnaire ; et en ce que, pour chaque modèle de compte-rendu de la bibliothèque, sont stockés dans la mémoire du serveur de traitement : au moins un jeu prédéfini d'une pluralité de règles de transformation d'une paire de question-réponse dudit compte-rendu vers une autre paire de question-réponse et/ou au moins une grille prédéfinie de sélection de paires de question-réponse parmi l'ensemble des paires de question-réponse dudit compte-rendu.

5. Procédé selon la revendication précédente, **caractérisé en ce que** chaque règle d'un jeu de règles de transformation associé à un modèle de compte-rendu est apte à remplacer, dans au moins une paire de question-réponse dudit compte-rendu, au moins une entrée donnée de la question et/ou de la réponse de ladite paire par une autre entrée prédéterminée.

6. Procédé selon l'une des revendications 4 ou 5, **caractérisé en ce qu'**une grille de sélection associée à un modèle de compte-rendu est apte à conserver ou supprimer chaque paire de question-réponse dudit compte-rendu en fonction de la question et/ou de la réponse de ladite paire.

7. Procédé selon l'une des revendications 3 à 6, **caractérisé en ce que**, pour chaque modèle de compte-rendu de la bibliothèque, sont stockés dans la mémoire du serveur de traitement au moins un jeu prédéfini d'une pluralité de règles de transformation et/ou au moins une grille prédéfinie de sélection associée à un premier niveau de compréhension et au moins un jeu prédéfini d'une pluralité de règles de transformation et/ou au moins une grille prédéfinie de sélection associé à un deuxième niveau de compréhension ; **en ce que** l'étape de génération automatique comporte :
a. une étape de sélection, par le serveur de traitement dans sa mémoire, de la grille de sélection et/ou du jeu de règles de transformation associés au modèle du premier compte-rendu et au premier niveau de compréhension et de la grille de sélection et/ou du jeu de règles de transformation associés au modèle du premier compte-rendu et au deuxième niveau de compréhension ;
b. une étape d'application au premier compte-rendu, par le serveur de traitement, de la grille de sélection et/ou du jeu de règles de transformation associés au premier niveau de compréhension pour générer le deuxième compte-rendu et de la grille de sélection et/ou du jeu de règles de transformation associés au deuxième niveau de compréhension pour générer un troisième compte-rendu ; et **en ce que** l'étape de transmission comporte la transmission, par le serveur de traitement, des premier, deuxième et troisième comptes-rendus au moins au premier terminal informatique.

8. Procédé selon l'une des revendications précédentes, **caractérisé en ce que** le serveur de traitement comporte une mémoire dans laquelle sont stockés au moins un lexique comportant une pluralité de mots-clés prédéterminés et au moins un dictionnaire associant à chaque mot-clé du lexique une définition dudit mot-clé, et **en ce que** l'étape de génération automatique du deuxième compte-rendu comporte :
a. une étape d'identification, par le serveur de traitement, d'un ou plusieurs mots-clés dans le premier compte-rendu à partir dudit lexique ;
b. pour chaque mot-clé identifié, une étape de sélection, par le serveur de traitement, d'une définition associée audit mot-clé identifié dans le dictionnaire ;
c. une étape d'insertion, par le secteur de traitement, de chaque définition sélectionnée dans le premier compte-rendu pour générer le deuxième compte-rendu.

9. Procédé selon la revendication précédente, dans lequel sont stockés dans la mémoire du serveur de traitement au moins un premier dictionnaire associé à un premier niveau de compréhension et au moins un deuxième dictionnaire associé à un deuxième niveau de compréhension, **caractérisé en ce que** l'étape de sélection comporte, pour chaque mot-clé identifié, la sélection d'une définition associée audit mot-clé dans le premier dictionnaire et dans le deuxième dictionnaire ; et **en ce que** l'étape d'insertion comporte l'insertion dans le premier compte-rendu de chaque définition sélectionnée dans le premier dictionnaire pour générer le deuxième compte-rendu et l'insertion dans le premier compte-rendu de chaque définition sélectionnée dans le deuxième dictionnaire pour générer un troisième compte-rendu ; et **en ce que** l'étape de transmission comporte la transmission, par le serveur de traitement, des premier, deuxième et troisième comptes-rendus au moins au premier terminal informatique.

10. Procédé selon l'une des revendications précédentes, **caractérisé en ce qu'**il comporte, ultérieurement à la génération de l'identifiant numérique :
a. une étape de saisie par le patient, via le deuxième terminal informatique, dudit identifiant numérique ;
b. à la suite de ladite saisie ; une étape de transmission par le serveur de traitement des premier et deuxième comptes-rendus au deuxième terminal informatique.

11. Procédé selon l'une des revendications précédentes, dans lequel le deuxième terminal informatique est équipé d'une application logicielle comportant une interface de sélection d'un niveau de compréhension de l'utilisateur du deuxième terminal informatique, **caractérisé en ce qu'**il comporte une étape ultérieure d'affichage, sur ladite application logicielle, du premier compte-rendu et/ou du deuxième compte-rendu selon le niveau de compréhension sélectionné par l'utilisateur du deuxième terminal informatique au moyen de ladite interface de sélection.

12. Procédé selon la revendication précédente, dans lequel ladite application logicielle comporte une interface de confirmation de compréhension par l'utilisateur du deuxième terminal informatique, **caractérisé en ce qu'**il comporte :
a. A la suite de l'affichage, sur ladite application logicielle, du premier compte-rendu et/ou du deuxième compte-rendu, une étape d'affichage, sur ladite application logicielle, de ladite interface de confirmation de compréhension ;
b. A la suite d'une saisie d'informations par l'utilisateur du deuxième terminal informatique, via ladite interface de confirmation de compréhension, la transmission desdites informations saisies au serveur de traitement et/ou au premier terminal informatique.
